# EUROPEAN PATENT APPLICATION

(11) **EP 2 356 901 A1**
(43) Date of publication of application: **17.08.2011**
(21) Application number: 10001476.0
(22) Date of filing: 12.02.2010
(51) Int. Cl.: A01K 67/033, C12N 15/63

(54) **Induction of gene expression in arthropods**

(71) Applicant: Fraunhofer-Gesellschaft zur Förderung der angewandten Forschung e.V., 80686 München (DE)
(72) Inventor: Rodrigues Makert dos Santos, Gustavo, 70499 Stuttgart (DE); Delaroque, Nicolas, 04317 Leipzig (DE); Szardenings, Michael, 38302 Wolfenbüttel (DE); Ulbert, Sebastian, 04275 Leipzig (DE); Giese, Matthias, 69123 Heidelberg (DE)
(74) Representative: Lahrtz, Fritz

(57) **Abstract**

The present invention relates to a method for the production of a gene encoded molecule in an arthropod *in vivo,* wherein a nucleic acid comprising a gene encoding said molecule is fed to the arthropod. Furthermore, the present invention relates to a method for the delivery of a purified nucleic acid to arthropod cells *in vivo,* wherein the purified nucleic acid is fed to the arthropod.

## Description

The present invention relates to the induction of gene expression in arthropods *in vivo,* especially to the induction of protein expression in insects *in vivo.*

Arthropods are a highly diverse group of animals comprising about 80% of all described living animal species. The most important classes of arthropods are hexapoda (which include insects), chelicerata (including spiders, mites and scorpions), crustacea and myriapoda (Brusca and Brusca 2003). An arthropod may be a pest in agriculture, an ectoparasite on animals, including human, an endoparasite in animals, including human, an epidemiologic vector, a pest destroying property by feeding or contamination or a pest endangering ecological balance. On the other hand, an arthropod may serve as native or agricultural animal feed, as nutrition for humans, as factor to balance an ecosystem or as a source of material susceptible of the industrial utilisation.

It is well-known in the art that insect cell cultures e.g. SF-9 or SF-21 cell cultures, are widely used in the art and are capable of producing considerable amounts of a gene encoded molecule, in particular a protein. In contrast, the production of a gene encoded molecule in an arthropod *in vivo* is still hampered by the problem to incorporate the required nucleic acid in the cells of said arthropod. There is still a considerable lack of capable techniques to enable a nucleic acid to enter into arthropod cells, which is different to the laborious and time consuming manipulation of germ cell lines.

Arthropods bear an impermeable barrier, in form of a chitin shield, all over their body surface and are therefore extensively protected from exogenous influences and extensively isolated from the molecular environment. Therefore, the application of a nucleic acid by methods regularly used in the art, such as the use of gene guns (Tang et al 1992), may fail or may be complicated. Furthermore, it is well-known in the art that the use of a nucleic acid, in particular the use of DNA and/or RNA, *in vivo* does mostly not lead to the desired result. It is well-known that an aforementioned nucleic acid is highly ineffective in *in vivo* applications in nearly all animal species (Wahren and Liu 2005). It is a common prejudice that this is also true for the use of nucleic acids in living arthropods.

There is a need for an efficient and simple method for inducing gene expression in arthropods *in vivo.*

In a first aspect, the present invention relates to a method for the production of a gene encoded molecule in an arthropod *in vivo,* wherein a nucleic acid comprising a gene encoding said molecule is fed to the arthropod.

In the context of the present invention, as shown in the example, it has been surprisingly found that by feeding a nucleic acid encoding a gene to an arthopod, it is possible to induce expression of said gene *in vivo.* Furthermore, it has been surprisingly found that the expressed gene product is also found in an ectoparasite living on the arthropod.

In the example section, species from three major orders of insects have been used as model organisms. In view of the close similarity to other arthropods, it is assumed that the results obtained for insects can also be extended to other arthropods.

Throughout the invention, the term "gene encoded molecule" refers to all molecules and combinations of molecules that may be generated by gene expression. Particularly, said term includes, but is not limited to a protein, or an RNA. According to the invention, the term "gene encoded molecule" does not include a siRNA or an antisense RNA or DNA.

In a preferred embodiment of the invention, the gene encoded molecule is a protein or a biologically active RNA. In the context of the present invention, the term "biologically active " denotes that the given molecule has a biological function.

Preferably, said biologically active RNA is selected from the group consisting of:
a. tRNA,
b. rRNA,
c. mRNA, and
d. double stranded RNA.

These molecules are known in the art.

Preferably, the RNA is a catalytic RNA capable of catalyzing chemical reactions.

In the most preferred embodiment, the gene encoded molecule is a protein. According to the invention, the term "protein" includes polypeptides composed of a consecutive sequence of amino acid moieties of all lengths including short peptides, protein domains, high-molecular weight proteins and proteins comprising independent similar or different subunits. The protein may be a protein naturally occurring in arthropods or may be a foreign protein, i.e. a protein which naturally does not occur in arthropods.

In a specific embodiment of the present invention, the gene encoded molecule is controlling the rate of the production of other molecules of interest in the arthropod. For example, the gene encoded molecule may be a protein, e.g. a hormone or a transcription factor, which induces the production of other proteins in the arthropod.

According to the invention, the production of the gene encoded molecule is induced by feeding to the arthropod a nucleic acid comprising a gene encoding said gene encoded molecule. Consequently, in the context of the present invention, the term "gene" refers to a DNA or RNA, preferably a DNA encoding said molecule. In this context, the "gene" may be a naturally occurring gene or a part thereof.

Said gene is according to the invention comprised in a nucleic acid. This nucleic acid is either a DNA or an RNA, depending on the gene. Preferably, the nucleic acid is a DNA.

Preferably, said nucleic acid is in the form of a purified nucleic acid, is contained in a bacterial cell, or an archeal cell, an eukaryotic cell or virus or virus-like particles or is complexed with a transfecting agent.

According to the invention, the term "purified nucleic acid" means that the nucleic acid has been purified, e.g. extracted, from the context where it has been produced, preferably a cell or a tissue, more preferably a bacterial cell. Methods for the purification of nucleic acids are known in the art, e.g. from Sambrook and Russel, 2001.

On the other hand, it is also included in the present invention that the nucleic acid may still be included in the cell where it has been produced, e.g. a bacterial or archeal cell or a eukaryotic cell. This means that the cell itself is fed to the arthropod. Furthermore, it is included in the present invention that the nucleic acid may be contained in a virus (e.g a baculovirus, an adenovirus, a vaccinia virus), a bacteriophage or a virus-like particle.

The nucleic acid may further be contained in a transfecting agent. As used herein, the term "transfecting agent" refers to molecular compositions that improve the cellular and systemic uptake of a nucleic acid. Said molecular compositions comprise but are not limited to liposome compositions and complexes and conjugates with fatty acids, synthetic polymers (in particular positively charged or hydrophobic polymers, e.g. polyethylene imine (PEI) and derivatives thereof), polysaccharides (e.g. chitosan), virus-like particles, proteins (in particular DNA- or RNA-interacting proteins and proteins comprising cell-penetrating peptides (CPPs)), protein transduction domains (PTDs) or antimicrobial peptides).

Said nucleic acid may preferably be either (i) plasmid DNA, (ii) linear DNA, (iii) circular DNA, (iv) single stranded DNA, (v) RNA, (vi) non natural DNA like molecules or (vii) a hybrid formed out of any of these molecules. These molecules are known in the art (see Sambrook and Russel, 2001).

In particular, commercially available plasmids can be used in the method of the invention, wherein the gene of interest is then cloned into said commercially available plasmid. In this context, it is preferred to use vectors that are not carrying additional genes such as antibiotic resistance genes, although also such vectors may be used.

Apart from the gene, the nucleic acid may contain further nucleic acid sequences, especially regulatory sequences like promoters or enhancers. Promoter and enhancer sequences are known in the art. Since the feeding of the nucleic acid results in the production of the gene encoded molecule, it is particularly preferred that the nucleic acid further contains a promoter active in said arthropod. Such a promoter may be a promoter which is active in a broad range of cells including e.g. the CMV promoter, or the TK promoter.

Furthermore, the promoter may be an arthropod, especially an insect specific promoter. Examples include the WSSVie1 promoter, a virus promoter from white spots syndrome virus which infest arthropods (Liu et al 2007), the B. mori (Bm) actin A3 promoter, the Apis m Actin promoter, and the Drosophila Hsp70 promoter.

In principle, the method of the present invention can be applied to all arthropods. In a preferred embodiment of the present invention, the arthropod may be but is not limited to an insect, an arachnid, a crustacean and a myriapodum.

In a further preferred embodiment of the present invention, the arthropod is an insect, in more preferred embodiments, the insect is a holometabolic insect or a hemimetabolic insect or the insect is from the order Hymenoptera, Coleoptera or Orthoptera. In the most preferred embodiment, the insect is a honey bee *Apis mellifera,* a mealworm *Tenebrio molitor,* or a Mediterranean field cricket *Gryllus bimaculatus.*

In another embodiment of the present invention, the arthropod is an arachnid, more preferred a mite or a spider.

In the context of the present invention, the nucleic acid is fed to the arthropod. In principle, every feed can be used that is accepted by the arthropod to be fed. This includes any kind material that is consumed orally by the arthropods, independent on whether it is natural feed, agricultural feed or laboratory feed and independent on whether it is consumed naturally or is administered by means of technical devices or is taken up casually. In a preferred embodiment, the feed that is used to induce the production of the gene encoded molecules in the arthropods is either a liquid feed comprising the nucleic acid, a dry feed mixed with a solution comprising the nucleic acid or a dry feed comprising the dry nucleic acid or any form of encapsulated nucleic acid (bacteria, archaea, viruses, virus-like particles, eukaryotic cells) in any of these formulations.

The method of the invention can be used for all purposes where the production of a gene encoded molecule in an arthropod is suitable and/or desirable.

This includes the use of the method of the invention to generate (i) a transfected arthropod or (ii) a transgenic arthropod, where at least part of the arthropod cells are transiently or stably transfected with the nucleic acid.

As used herein, the term "transgenic arthropod" refers to an arthropod in which at least in some of the cells the nucleic acid has inserted into the genome in a stable thus irreversible way or to a progeny of said arthropod. The term "transiently transfected", in contrast, refers to a situation where no stable integration of the nucleic acid into the genome has occurred.

Consequently, the method of the invention may be used to generate a cell culture of transiently transfected or transgenic arthropod cells, e.g. by isolating the cells from the fed arthropod.

Furthermore, the method of the invention may be used for the production of a recombinant protein, especially a pesticide, a regulatory protein or a vaccine antigen. The production of a pesticide is especially suitable in cases where the arthropod, in particular an insect, is a pest.

Throughout the invention, the term "vaccine" refers to every gene encoded molecule, in particular a protein, that can provoke a cellular or a humoral immune response in a subject. The arthropod expressing said antigen is eaten by an animal (e.g. small mammals, birds or fish), hence inducing an immune response against said antigen.

Additionally, the method of the invention may be used for the production of an arthropod useful in pharmacological screenings. For such screenings the gene product may be the target of drug substances that are preferably tested in a living animal. Most preferably the gene product allows direct monitoring of the actions of insectizides or other agents that change the cellular status. Such monitoring systems, which are well known in the art, can otherwise only be used in certain cases in species accessible to germ line manipulations.

In particular, the method of the invention may be used for the production of an arthropod useful for vaccination of insectivores or for protecting insectivores against ectoparasites. As used herein, the term "insectivore" refers to all kinds of animals, including human, that feed/eat regularly, sporadically or exceptionally insects or parts thereof, larvae of insects or parts thereof, pupals of insects or part thereof, cocoons of insects or part thereof, eggs of insects or part thereof, imago of insects or part thereof or metabolic products of the insects, larvae, pupal, cocoon or eggs (e.g. silk or other cocoon materials or chitin or chitosan) or products thereof.

Similarly, the arthropod may be a crustacean which is eaten by all kinds of animals, including human, that feed/eat regularly, sporadically or exceptionally crustacea or parts thereof.

The usage of crustaceans, similar to insects, opens in addition of the feeding of insects the possibilities to vaccinate fish, which would be extremely useful for commercial fish breeding.

Furthermore, the method of the invention may be used for protecting the arthropod against pest influences. As used herein, the term "pest influences" refers to all influences that cause a decrease in the viability or a decrease of the health of the arthropod, in particular but not limited to natural and synthetic toxins and pathogens, in particular viruses, bacteria, moulds, protozoa, other arthropods and other animals, plants and fungi.

In particular, the method of the present invention may be used for protecting the arthropod against an ectoparasite of the arthropod. In particular, honey bee *Apis mellifera* can be protected against the ectoparasite *Varroa destructor,* as shown in the present examples, because by feeding a honey bee, it is possible to obtain the presence of the gene encoded molecule also in the ectoparasite *Varroa destructor.* In a preferred embodiment, said gene encoded molecule is an acarizide which is harmful to the parasitizing mite *Varroa.* This is of enormous economic significance, since the damages caused by said ectoparasite are very high. It is estimated that losses due to the weakness and collapse of bee colonies worldwide reach several billion US$ per year.

In another aspect, the present invention relates to a method for the delivery of a purified nucleic acid to arthropod cell *in vivo,* wherein the purified nucleic acid is fed to the arthropod.

As shown in the examples, it is possible to induce gene expression simply by feeding a purified nucleic acid to an arthropod. This provides a very simple method of gene delivery.

With respect to this aspect of the invention, all embodiments disclosed above with respect to the nucleic aid, the arthropod, the way of feeding as well as of the use of the method of the invention do also apply.

In a preferred embodiment of said aspect of the invention, said nucleic acid encodes an siRNA or an antisense RNA or is an siRNA or antisense RNA. In this case, the siRNA or antisense molecule is produced in the arthropod by feeding the arthropod with the nucleic acid. In this context, the nucleic acid is preferably a plasmid encoding the siRNA and further containing promoter sequences enabling the production of the siRNA. Alternatively, the nucleic acid may be the siRNA or antisense molecule itself. In this case, the siRNA or antisense molecule is preferably protected, e.g. by methylation or by the use of nucleic acid analogues, such as Peptide nucleic acids (PNAs), morpholinos or locked nucleic acids (LNAs), in order to prevent degradation of the molecule.

The invention is further explained by the following examples and figures, which are intended to illustrate, but not to limit the scope of the present invention.

### Brief description of the Figures

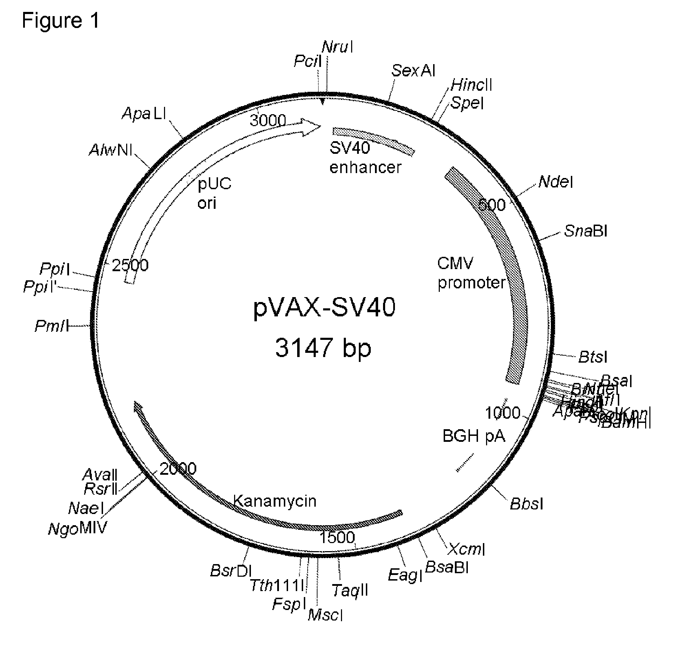

**Figure 1****.** pVAX-SV40 plasmid.

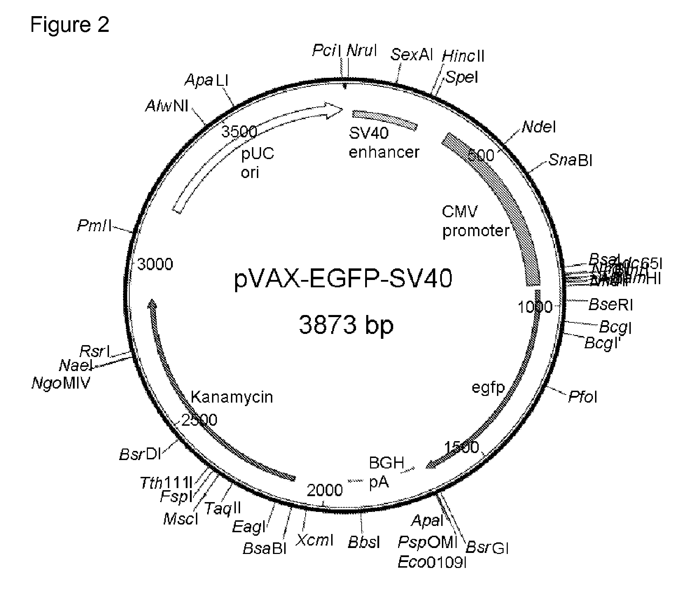

**Figure 2****.** pVAX-EGFP-SV40 plasmid

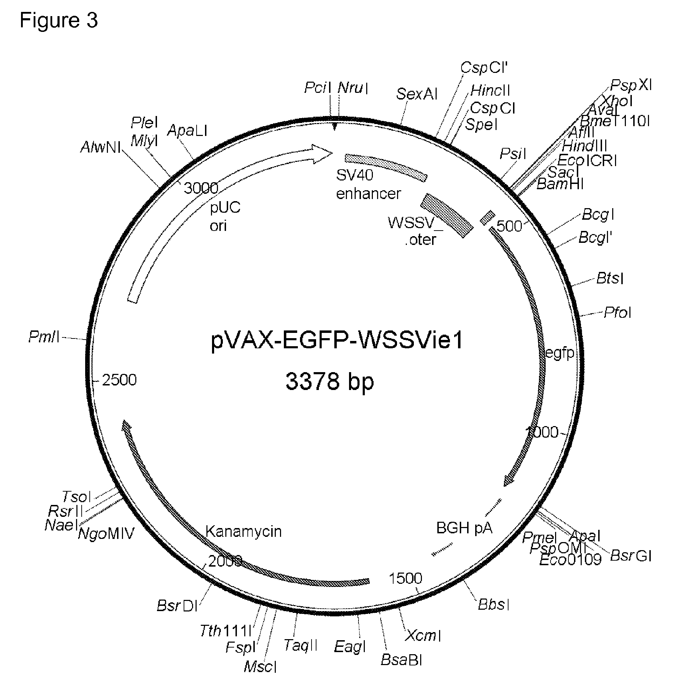

**Figure 3****.** pVAX-EGFP-WSSVie1 plasmid.

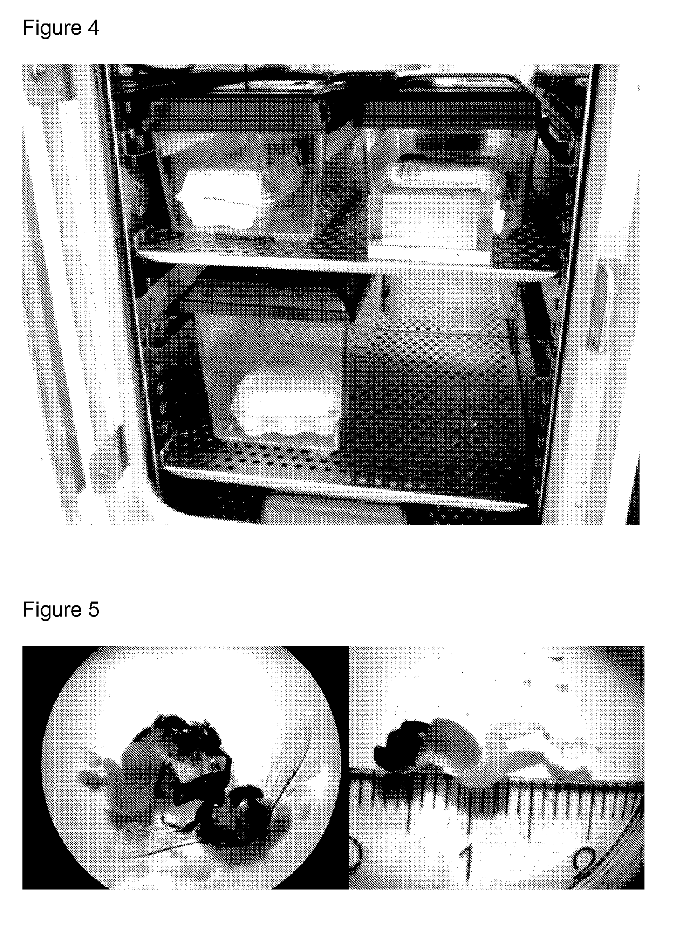

**Figure 4****.** Brood combs of honey bee *Apis mellifera* containing brood in the late pupal stages and 1 day old adults were maintained in an incubator at 32°C for several weeks (3-4 weeks).
**Figure 5****.** Muscle and gut preparation of honey bee *Apis mellifera* which were treated with oral application of DNA-plasmids. These bees were maintained in an incubator at 32°C for 3-4 weeks. The analysis of organs and pictures were made with a Canon Power Shot 66 camera installed in a Carl Zeiss Stemi 2000-C microscope.

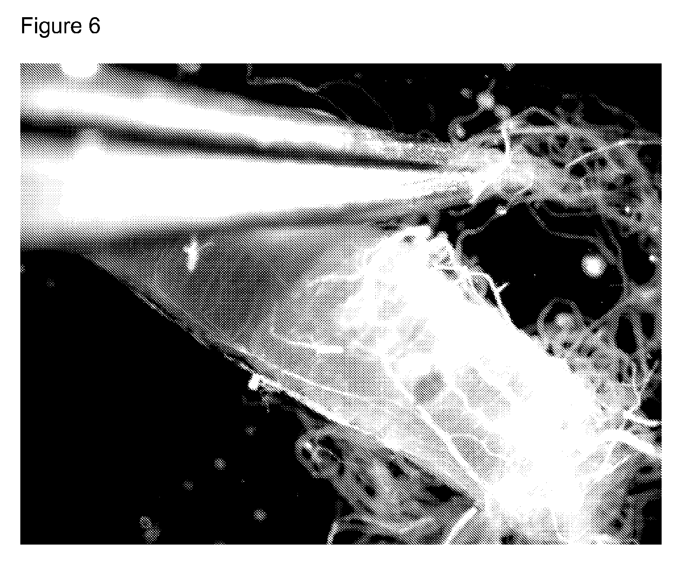

**Figure 6****.** Malpighian tubule system of honey bee *Apis mellifera* was prepared after treatment with oral application of DNA-plasmids and maintained in an incubator at 32°C.
The analysis of organs and pictures were made with a Canon Power Shot 66 camera installed in a Carl Zeiss Stemi 2000-C microscope.

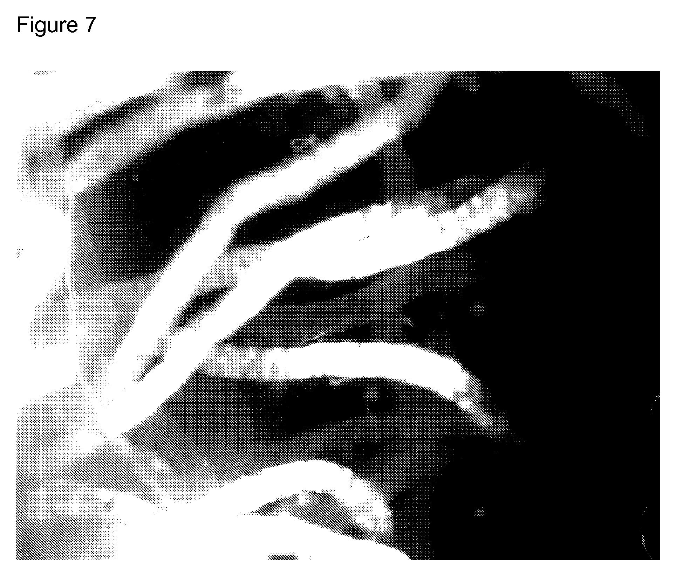

**Figure 7****.** Malpighian tubule system of honey bee *Apis mellifera* prepared after treatment with oral application of EGFP-plasmid. The analysis and pictures were made with a Leica DFC 350 FX camera installed in a Leica CTR 4000 fluorescent microscope. The magnification was 100 x, the exposure time was 70 ms, gamma was 0.6 and gain 1.0X.

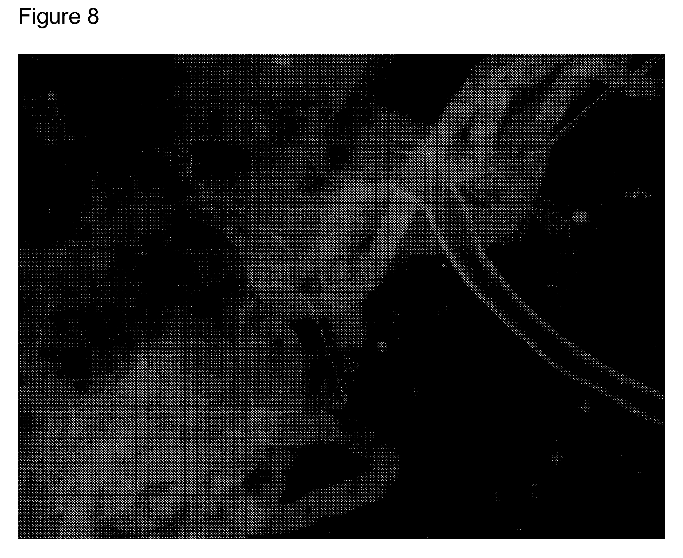

**Figure 8****.** Malpighian tubule system of negative control honey bee *Apis mellifera* prepared after oral application of DNA. The analysis and pictures were made with a Leica DFC 350 FX camera installed in a Leica CTR 4000 fluorescent microscope. The magnification was 100 x, the exposure time was 70 ms, gamma was 0.6 and gain 1.0X.

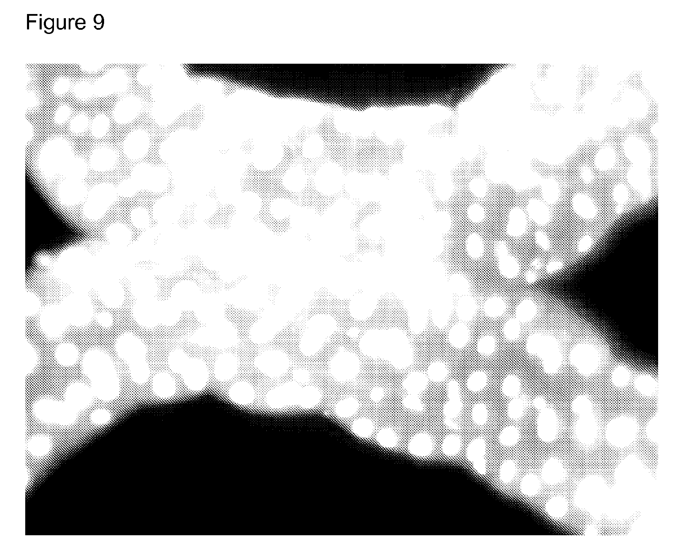

**Figure 9****.** Nuclei detection in Malpighian tubule system of honey bee *Apis mellifera* prepared after treatment with Dapi nucleic acid staining and fluorescent mounting medium. The analysis and pictures were made with a Leica DFC 350 FX camera installed in a Leica CTR 4000 fluorescent microscope. The magnification was 400 x, the exposure time was 70 ms, gamma was 0.6 and gain 1.0X.

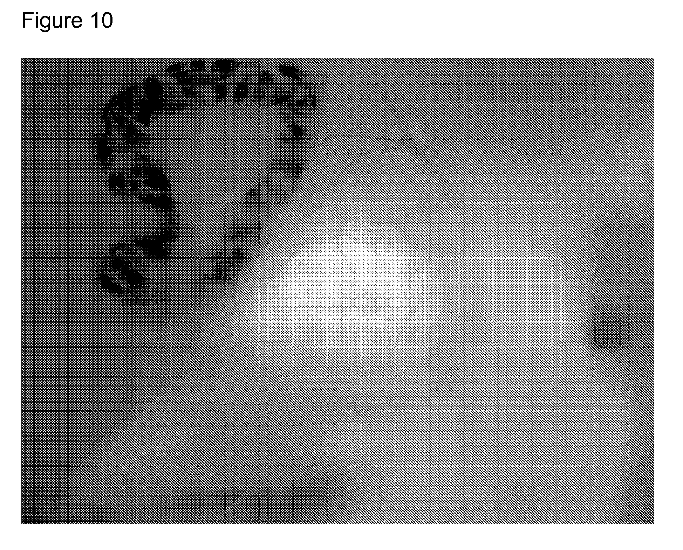

**Figure 10****.** Intestine of negative control mealworm *Tenebrio molitor* prepared after treatment with oral application of EGFP-plasmid. The analysis and pictures were made with a Leica DFC 350 FX camera installed in a Leica CTR 4000 fluorescent microscope. The magnification was 100 x, the exposure time was 200 ms, gamma was 0.6 and gain 1.0X.

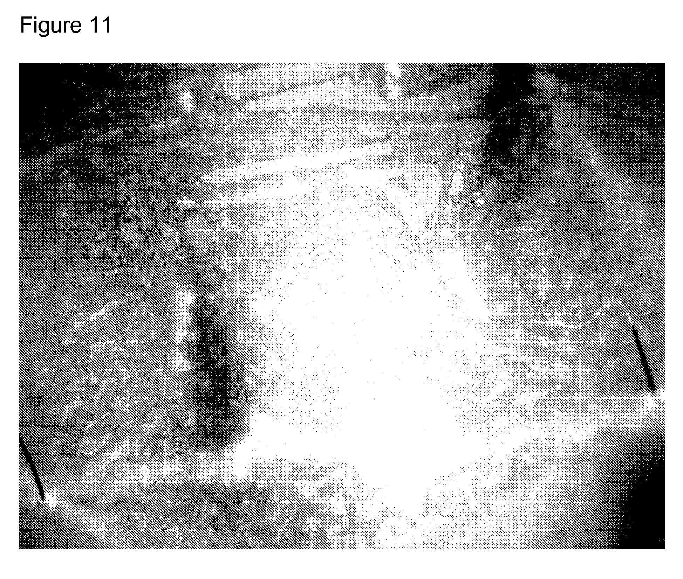

**Figure 11****.** Intestine of negative control mealworm *Tenebrio molitor* prepared after treatment with oral application. The analysis and pictures were made with a Leica DFC 350 FX camera installed in a Leica CTR 4000 fluorescent microscope. The magnification was 100 x, the exposure time was 200 ms, gamma was 0.6 and gain 1.0x.

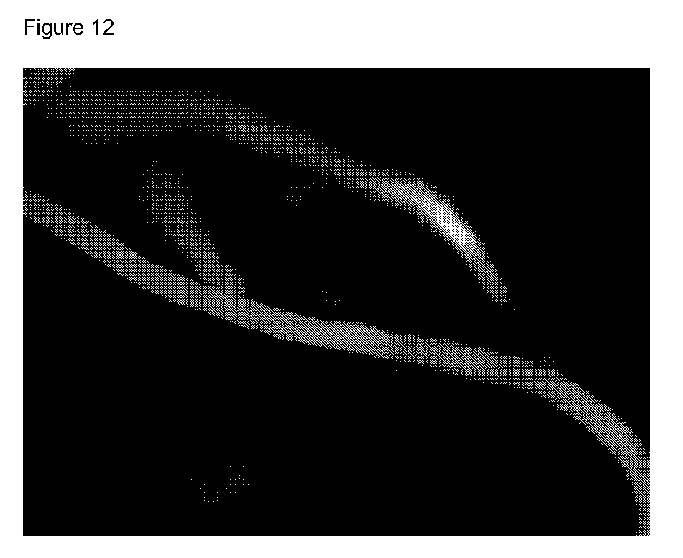

**Figure 12****.** Malpighian tubule system of cricket *Gryllus bimaculatus* prepared after treatment with oral application of EGFP-plasmid. The analysis and pictures were made with a Leica DFC 350 FX camera installed in a Leica CTR 4000 fluorescent microscope. The magnification was 100 x, the exposure time was 200 ms, gamma was 0.6 and gain 1.0X.

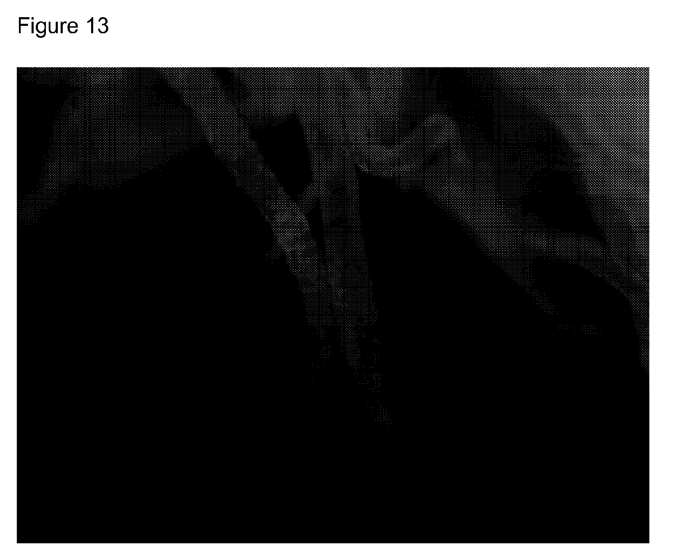

**Figure 13****.** Malpighian tubule system of negative control cricket *Gryllus bimaculatus* prepared after treatment with oral application. The analysis and pictures were made with a Leica DFC 350 FX camera installed in a Leica CTR 4000 fluorescent microscope. The magnification was 100 x, the exposure time was 200 ms, gamma was 0.6 and gain 1.0X.

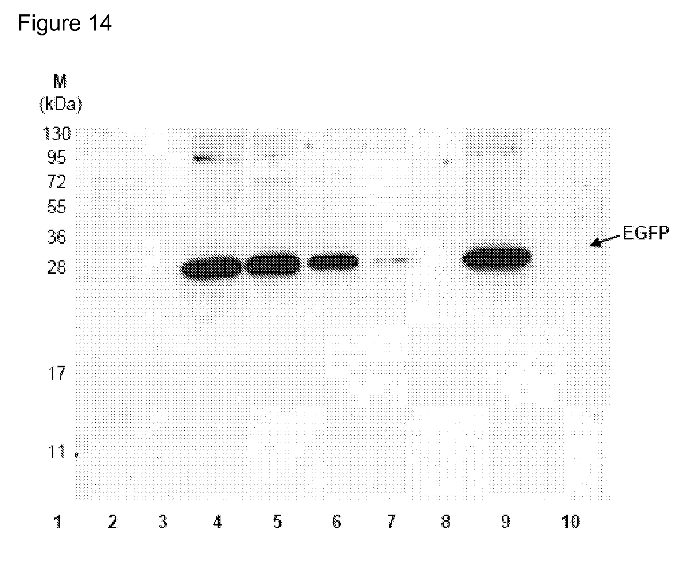

**Figure 14****.** Western blots from *Tenebrio molitor* performed after oral application of DNA. The lane 1 is the page ruler plus prestained protein ladder. Lane 2 is the negative control (oral application with pVAX-SV40). Lane 3 is empty. Lanes 4 to 6 show EGFP from different animals with a dilution of 1:10. Lane 7 shows EGFP from the same sample from lane 6, but with a dilution of 1:50. Lane 8 and 10 are empty. Lane 9 shows the positive control. All samples were diluted 1:5 with 15% mercaptoethanol loading dye buffer.

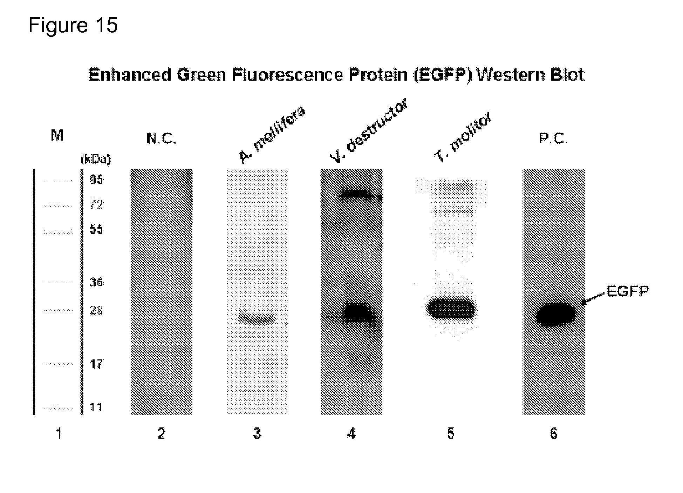

**Figure 15****.** Western blots developed from insect cells after oral application of DNA. The lane 1 shows the page ruler plus prestained protein ladder. Lane 2 shows the negative control (oral application with pVAX-SV40). Lane 3 shows EGFP from *Apis mellifera* oral application and lane 4 from *Varroa destructor* samples. Lane 5 shows GFP from *Tenebrio molitor.* Lane 6 shows the positive control. All samples were diluted 1:5 with 15% mercaptoethanol loading dye buffer.

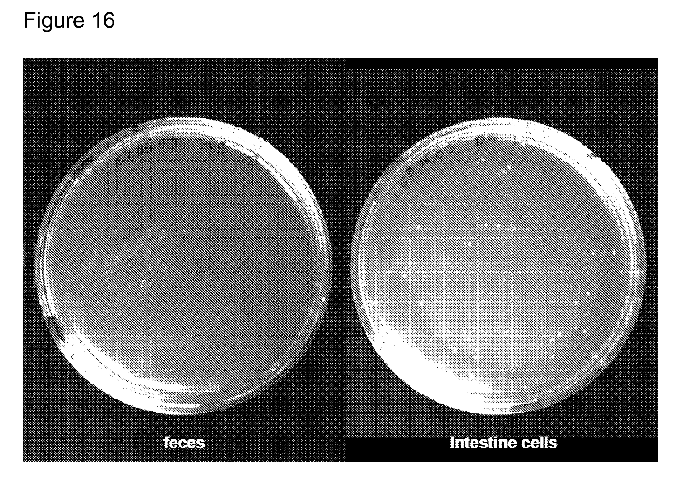

**Figure 16****.** Contamination tests developed with electrocompetent BL21 cells transformation from insect intestine cells and feces extracts after oral application treatment. 1 µl feces or gut extract was used for the transformation. BL21 cells (30 µl) were transformed through electroporation (Voltage 1800 V, Capacitance 25 µF, Resistance, 200 Ω), with 1-mm cuvettes and a GenePulser Xcell machine (Bio-Rad).

### Example

The following example *iter alia* demonstrates that feeding a nucleic acid to an arthropod is capable to induce the production of gene encoded molecules in said arthropod.

### Abstract

In order to show the capability of the method to feed functional nucleic acid and thereby induce gene expression, a nucleic acid was used in different species of arthropods and the production of the gene encoded molecule enhanced green fluorescent protein (EGFP) was induced in the said arthropods. The fluorescence was detected in different organs and tissues of the arthropod. In addition, Western blot analysis was performed from samples of the arthropod tissues.

### Introduction

It is well-known in the art that insect cell cultures are capable to produce considerable amounts of a gene encoded molecule, in particular a protein. In contrast, the production of a gene encoded molecule in an arthropod *in vivo* is still hampered by the problem to incorporate the required nucleic acid in the cells of said arthropod. There is still a considerable lack of capable techniques to enable a nucleic acid to enter into arthropod cells.

Arthropods bear an impermeable barrier, in form of a chitin shield, all over their body surface and are therefore extensively protected from exogenous influences and extensively isolated from the molecular environment. Therefore, the application of a nucleic acid by methods regularly used in the art, such as the use of gene guns, may fail or may be complicated. Furthermore, it is well-known in the art that the use of a nucleic acid, in particular the use of DNA and/or RNA, *in vivo* does mostly not lead to the desired result. It is well-known that an aforementioned nucleic acid is highly ineffective in *in vivo* applications in nearly all animal species. It is a common prejudice that this is also true for the use of nucleic acids in living arthropods.

In summary, the problem of the application of a nucleic acid in arthropods to induce the production of a gene encoded molecule existed for a long time and, so far, no capable solution was disclosed in the art, other than laborious and time consuming embryonic manipulation.

In the present invention, we introduced a capable method for the use of a nucleic acid in an arthropod *in vivo* and induce the production of the gene encoded molecule EGFP. The nucleic acid DNA encoding for EGFP was fed to the arthropod, whereupon EGFP was induced in different organs of the arthropod. We thereby circumvented the chitin shield barrier.

### Methods

### Antibodies and reagents

The plasmids used in this invention are based the on pVAX1 plasmid purchased from Invitrogen. The colonies of honey bee *Apis mellifera* were obtained from a beekeeper in Leipzig, Germany. The external parasite mite *Varroa destructor* was found on the collected bees. The first antibody for Western blotting was purchased from Cell Signaling. The second antibody (the polyclonal goat anti-rabbit immunoglobulins (IgG)/HRP) was obtained from Dako Denmark A/S. Electroporation cuvettes (1 mm) and the GenePulser Xcell machine were supplied by Bio-Rad. The page ruler plus prestained protein ladder was purchased from Fermentas.

### Plasmid construction

Three plasmids were constructed (Figures 1, 2 and 3). The first one (pVAX-SV40) was used as negative control (Figure 1). This plasmid is based on commercially available pVAX1 and was modified by the insertion of a 72bp SV40 enhancer element. The second plasmid pVAX-EGFP-SV40 (Figure 2) was generated by inserting the coding sequence for EGFP in the MCS of pVAX-SV40, hence downstream of the CMV promoter. The third plasmid was pVAX-EGFP-WSSVie1 (Figure 3). Here, the virus promoter from white spots syndrom virus (Liu et al. 2007) was used to replace the CMV promoter.

### DNA-plasmid oral application

The experiments were performed on 10 colonies of honey bee *Apis mellifera.* Brood combs containing brood in the late pupal stages and 1 day old adults were removed from colonies and maintained 3-4 weeks in an incubator at 32°C (Figure 4). The external parasite mite *Varroa destructor* was found on the collected bees and was also used for the oral application experiments. In addition, we tested the oral application with two other insect species: the mealworm *Tenebrio molitor* (coleoptera), a holometabolic insect, and in the Mediterranean field cricket *Gryllus bimaculatus,* a hemimetabolic insect. All insect species were treated with the three plasmids (Figures 1, 2 and 3). Dilution to 500 ng/µl plasmid was made with water (for cricket *Gryllus bimaculatus*) and glucose-water (1:1) (for the honey bee *Apis mellifera*)*,* respectively. The cricket *Gryllus bimaculatus* was fed with dried feed too. The cricket food was prepared based on the method described by Ibler et al. (Ibler et al., 2009). The crickets were fed with a dried mixture of 2.4 g cricket food + 1 ml [1mg/ml] plasmid. The food for *Tenebrio molitor* consisted of a dried mixture of 2.4 g meal + 1ml [1mg/ml] plasmid.

### Fluorescence microscopy

After the treatment of oral application, different insect tissues were prepared (Figures 5 and 6) under a Carl Zeiss Stemi 2000-C microscope with a Canon Power Shot 66 camera. Intestine and Malpighian tubule system were analyzed by a Leica CTR 4000 fluorescent microscope with a Leica DFC 350 FX camera.

### Western blots

In order to confirm the presence of green fluorescent protein western blots from gut and muscle of these insects were performed. The first antibody for the EGFP detection was diluted 1:2000 in 5% milk PBS-Tween solution and incorporated for 1.5 h. The second antibody was the polyclonal goat anti-rabbit immunoglobulins (IgG)/HRP. This second antibody was diluted 1:2000 in 5% milk PBS-Tween solution and incorporated for 1 h.

### Contamination tests

To analyze whether the plasmids fed to the insects would leave the animal, we tested for the presence of functional plasmids in the feces. The tests were performed by transformation assays in highly competent E. coli cells. 1 µl of feces and intestine cells, respectively, was used for the transformation with electrocompetent BL21 cells. BL21 cells (30 µl) were transformed through electroporation with 1-mm cuvettes and a GenePulser Xcell machine (Bio-Rad). After transformation 300 µl of SOC medium was added. 100 µl of bacteria were spread onto LB plates containing 50 µg/µl Kanamycin. The plates were placed at 37°C overnight.

### Results

### 1. Induction of the production of EGFP in the honey bee Apis mellifera

EGFP protein could be microscopically detected in the Malphigian tubule system of a honey bee *Apis mellifera* that had been treated with the oral application of the plasmid pVAX-EGFP-SV40 (Figures 7 and 9). In contrast, a bee that was treated with the negative control, the plasmid pVAX-SV40, did not show any EGFP protein in its Malphigian tubule system (Figure 8). The same result was found in a sample from a muscle of a bee that had been treated with the plasmid pVAX-EGFP-SV40. In the latter case, the EGFP was detected by Western blot analysis (Figure 15, lane 3).

### 2. Induction of the production of EGFP in the mealworm Tenebrio molitor

EGFP protein could be microscopically detected in the intestinal tissue of a mealworm *Tenebrio molitor* that had been treated by the oral application of the plasmid pVAX-EGFP-SV40 (Figure 10). In contrast, a mealworm that was treated with the negative control, the plasmid pVAX-SV40, did not show any EGFP protein in its intestinal tissue (Figure 11). The same result was found in a sample from a mealworm that had been treated with the oral application of the plasmids pVAX-EGFP-SV40 and pVAX-SV40. EGFP was only detected when the mealworm was treated with the plasmid pVAX-EGFP-SV40 (Figure 14 and 15, lane 5). When the mealworm was treated with the negative control pVAX-SV40, no EGFP could be detected (Figure 14).

### 3. Induction of the production of EGFP in the cricket Gryllus bimaculatus

EGFP protein could be microscopically detected in the Malphigian tubule system of cricket *Gryllus bimaculatus* that had been treated with the oral application of the plasmid pVAX-EGFP-SV40 (Figure 12). In contrast, a cricket that was treated with the negative control, the plasmid pVAX-SV40, did not show any EGFP protein in its Malphigian tubule system (Figure 13).

### 4. Induction of the production of EGFP in the mite Varroa destructor

EGFP protein could be detected in the mite *Varroa destructor* parasiteizing on a honey bee *Apis mellifera* that was orally treated with the plasmid pVAX-EGFP-SV40. The detection was performed by Western blot (Figure 15, lane 4).

### 5. Contamination tests

The transformation with electrocompetent cells indicates that the functional plasmid is present in the intestine cells but is absent in the feces (Figure 16). This results indicates that plasmids which are outside of intestine cells were digested and would not spread into nature.

### 6. Detection Limit

To determine the detection limit of the contamination test, bee feces from untreated bees was spiked with 1 ng of plasmid DNA. In addition, naked DNA was used (1 ng, 100 pg, 10 pg). Transformations were performed as described above.

In the transformation experiment with 10 pg of plasmid there were 4 colonies formed. Based on this result it was possible to calculate the number of molecules. The transformation with electrocompetent cells therefore had a detection limit of about 200,000 molecules.

### Discussion

In this study, we provide formal experimental evidence that it is possible to induce the production of a gene encoded molecule in a living arthropod by feeding a nucleic acid containing the respective gene. In the presented examples an EGFP protein was expressed in different organs and tissues of different arthropods. The employment of a negative control proved that the induction of gene expression was dependent on the sequence identity of the nucleic acid. Furthermore, it was demonstrated that the employed plasmid was entirely inactivated while passing through the gastrointestinal tract of an arthropod. The latter indicates that a nucleic acid is not spread in nature.

Summarised, the present invention demonstrates the usability of a method based on feeding of arthropods with feed comprising a nucleic acid comprising a gene encoding a molecule of interest in numerous technical fields. Said method based on feeding may be conducted easily by a person skilled in the art. In a first step, the nucleic acid may be obtained by standard genetic engineering methods well-known in the art. Subsequently, the plainness of the feeding procedure easily allows the incorporation of the nucleic acid into an arthropod of interest.

Consequently, the present invention represents a capable technical solution to overcome the problem of usability of nucleic acids in living arthropods and inducing production of a molecule of interest therein. Said problem existed for a long time and its solution may allow the use of the presented invention in numerous applications. Furthermore, the prejudice that the *in vivo* use of nucleic acids administered orally did not result in an induction of the production of molecules of interest was overcome by aforementioned experiments.

### References

Brusca R.C. & Brusca G.J. (2003) Invertebrates. Sinauer Associates, Sunderland, MA.
Ibler B., Makert G. R. and Lorenz M. W. (2009) Larval and adult development and organisation of a systemic breeding of the Mediterranean field cricket (Gryllus bimaculatus de Geer, 1773). Zool.Garten 78: 81-101.
Liu W. J., Chang Y. S., Wang A. H., Kou G. H. and Lo C. F. (2007) White spot syndrome virus annexes a shrimp STAT to enhance expression of the immediateearly gene iel. J Virol. 81: 1461-1471.
Sambrook J. and Russel D. W. (2001) Molecular Cloning - Laboratory Manuals, 3rd edition, Cold Spring Harbour Laboratory Press, New York, USA.
Tang D.C., Devit M. and Johnston S.A. (1992) Genetic Immunization Is A Simple Method for Eliciting An Immune-Response. Nature 356, 152-154.
Wahren B. and Liu M. (2005) DNA Vaccines - An Overview. In: DNA Vaccination and Immunotherapy, pp. 1-6. Wiley-VCH Verlag GmbH & Co. KGaA, Weinheim, Germany.

## Claims

1. A method for the production of a gene encoded molecule in an arthropod *in vivo*, wherein a nucleic acid comprising a gene encoding said molecule is fed to the arthropod.

2. The method according to claim 1, wherein said gene encoded molecule is a protein or a biologically active RNA.

3. The method of claim 2, wherein said biologically active RNA is selected from the group consisting of:
a. tRNA,
b. rRNA,
c. mRNA, and
d. double stranded RNA.

4. The method according to any of claims 1 to 3, wherein the gene encoded molecule is a protein.

5. The method according to any of claims 1 to 3, wherein the gene encoded molecule is controlling the rate of the production of other molecules of interest in the anthropode.

6. The method according to any of claims 1 or 5, wherein said nucleic acid is in the form of a purified nucleic acid, is contained in a bacterial cell or virus or is complexed with a transfecting agent.

7. The method according to any of claims 1 to 6, wherein said nucleic acid is either (i) plasmid DNA, (ii) linear DNA, (iii) circular DNA, (iv) single stranded DNA, (v) RNA, (vi) non natural DNA like molecules or (vii) a hybrid formed out of any of these molecules.

8. The method of any one of claims 1 to 7, wherein the nucleic acid is a DNA.

9. The method according to any of claims 1 to 8, wherein said nucleic acid comprises a promoter active in said arthropod.

10. The method according to any of claims 1 to 9, wherein the arthropod is selected from the group consisting of an insect, an arachnid, a crustacean and a myriapodium.

11. The method according to claim 10, wherein the arthropod is an insect.

12. The method according to claim 11, wherein the insect is a holometabolic insect or a hemimetabolic insect.

13. The method according to claim 11 where the insect is from the order Hymenoptera, Coleoptera or Orthoptera.

14. The method according to any of claims 9 to 12, wherein said insect is a honey bee *Apis mellifera,* a mealworm *Tenebrio molitor,* or a Mediterranean field cricket *Gryllus bimaculatus.*

15. The method according to any of claims 1 to 14, wherein the feed is
a. a liquid feed comprising the nucleic acid;
b. a dry feed mixed with a solution comprising the nucleic acid; or
c. a dry feed comprising the dry nucleic acid.

16. The method according to any of claims 1 to 15, wherein said method is used to generate (i) a transfected arthropod or (ii) a transgenic arthropod, where at least part of the arthropod cells are transiently or stably transfected with the nucleic acid.

17. The method according to claim 15, wherein said method is used to generate a cell culture of transiently transfected or transgenic arthropod cells.

18. The method of any of claims 1 to 17, wherein said method is used for the production of a recombinant protein, especially a pesticide, a regulatory protein or a vaccine antigen.

19. The method of any of claims 1 to 18, wherein said method is used for the production of an arthropod useful in pharmacological screenings.

20. The method of any of claims 1 to 19, wherein said method is used for the production of an arthropod useful for vaccination of insectivores or for protecting insectivores against ectoparasites.

21. The method of any of claims 1 to 19, wherein said method is used for protecting the arthropod against pest influences.

22. The method of any of claims 1 to 19, wherein said method is used for protecting the arthropod against an ectoparasite of the arthropod.

23. The method of claim 22, wherein said ectoparasite is *Varroa destructor* and said arthropod is a honey bee *Apis mellifera.*

24. A method for the delivery of a purified nucleic acid to arthropod cells *in vivo,* wherein the purified nucleic acid is fed to the arthropod.

25. The method according to claim 24, with the features as defined in any of claims 6 to 23.

26. The method according to claim 25, wherein said nucleic acid encodes an siRNA or an antisense RNA or is an siRNA or antisense RNA.
